# EUROPEAN PATENT SPECIFICATION

(11) **EP 2 483 424 B1**
(45) Date of publication and mention of the grant of the patent: **04.12.2013**
(21) Application number: 10798597.0
(22) Date of filing: 01.10.2010
(51) Int. Cl.: C12Q 1/68

(54) **METHOD FOR THE DIAGNOSIS/PROGNOSIS OF AGE-RELATED MACULAR DEGENERATION**
VERFAHREN ZUR DIAGNOSE/PROGNOSE VON ALTERSBEDINGTER MAKULADEGENERATION
PROCÉDÉ DE DIAGNOSTIC/PRONOSTIC DE LA DÉGÉNÉRESCENCE MACULAIRE LIÉE À L'ÂGE

(30) Priority: 02.10.2009 US 247982 P
(43) Date of publication of application: 08.08.2012
(73) Proprietor: INSERM - Institut National de la Santé et de la Recherche Médicale, 75013 Paris (FR)
(72) Inventor: SOUIED, Eric, F-94000 Creteil (FR); ZERBIB, Jennyfer, F-94000 Creteil (FR); ROZET, Jean-Michel, F-75743 Paris Cedex 15 (FR)
(74) Representative: Hirsch, Denise Marie
(86) International application number: PCT/IB2010/003018
(87) International publication number: WO 2011/039650

(56) References cited:
- WO-A1-2009/059319
- WO-A2-2009/097418
- US-A1- 2003 162 758
- PROVOST ALEXANDRA C ET AL: "Morphologic and electroretinographic phenotype of SR-BI knockout mice after a long-term atherogenic diet.", INVESTIGATIVE OPHTHALMOLOGY & VISUAL SCIENCE, vol. 50, no. 8, August 2009 (2009-08), pages 3931-3942, XP002621511, ISSN: 1552-5783
- LEVEZIEL NICOLAS ET AL: "PLEKHA1-LOC387715-HTRA1 polymorphisms and exudative age-related macular degeneration in the French population.", MOLECULAR VISION, vol. 13, 2007, pages 2153-2159, XP002621512, ISSN: 1090-0535
- CONSTANTINEAU J A ET AL: "THE SYNONYMOUS VARIANT (RS5888/A350) WITHIN THE SCAVENGER RECEPTOR, CLASS B, TYPE I GENE IS ASSOCIATED WITH LOWER SR-BI PROTEIN EXPRESSION", ATHEROSCLEROSIS SUPPLEMENTS, vol. 9, no. 1, 1 May 2008 (2008-05-01), page 34, XP022651631, ELSEVIER ISSN: 1567-5688, DOI: 10.1016/S1567-5688(08)70129-6 [retrieved on 2008-05-01]
- ZERBIB JENNYFER ET AL: "rs5888 variant of SCARB1 gene is a possible susceptibility factor for age-related macular degeneration.", PLOS ONE, vol. 4, no. 10, E7341, October 2009 (2009-10), pages 1-6, XP002621513, ISSN: 1932-6203

## Description

### FIELD OF THE INVENTION

The invention is in the field of age-related macular degeneration (AMD) detection, diagnosis, prognosis and therapy. In particular, the invention relates to a specific single nucleotide polymorphism (SNPs) in the human genome and its association with age-related macular degeneration (AMD).

### BACKGROUND OF THE INVENTION

Age-related macular degeneration (AMD) is the most common cause of irreversible vision loss in the elderly population in Europe and United States. Identification of risk factors is of major importance for the understanding of the origins of the disorder and for establishing strategies to prevent AMD. Risk factors for AMD are both environmental and genetic. Over the past few years, several single nucleotide polymorphisms (SNPs) have been associated with AMD, including variants in the CFH and ARMS2 genes. The association between these polymorphisms and AMD risk suggests a pathway of inflammation and oxidation in AMD. Besides these pathways, several lines of evidence suggest a strong role of antioxidant micronutrients (xanthophylls, vitamin E and vitamin C) and lipids in AMD.

Nonetheless, up to this point, no single biomarker is sufficiently specific to provide adequate clinical utility for the diagnosis of age-related macular degeneration (AMD) in an individual subject.

Therefore, there is a need for identifying other factors that provide a more accurate diagnosis/prognosis of age-related macular degeneration (AMD).

The development of molecular biological techniques and the primary completion of the human genome project have enabled the detection of genetic variations that may be directly or indirectly related to age-related macular degeneration (AMD). Thus, the invention aims to provide a novel method for the diagnosis/prognosis of age-related macular degeneration (AMD) using a genetic factor.

### SUMMARY OF THE INVENTION

The present invention relates to a method for predicting risk of age related maculopathy or age-related macular degeneration, in a subject, said method comprising detecting in a sample obtained from said subject the single nuclotide polymorphism rs5888 in the SCARB1 gene, wherein the presence of the minor allele T of said polymorphism is indicative of increased risk of age related maculopathy or age-related macular degeneration.

### DETAILED DESCRIPTION OF THE INVENTION

### Definitions

Throughout the specification, several terms are employed and are defined in the following paragraphs.

"Age-related macular degeneration (AMD)" is defined as an ocular disease leading to loss of central vision in the elderly. The disorder is characterized by both primary and secondary damage of macular retinal pigment epithelial (RPE) cells resulting in formation of drusen (yellow deposits that form between the RPE and the underlying Bruch's membrane), formation of new choroidal vessel membranes termed choroidal neovascularisation (CNV) and atrophy of photoreceptors and choriocapillaris layer of the choroid. Age-related macular degeneration begins with characteristic yellow deposits in the macula (central area of the retina, which provides detailed central vision, called the fovea) called. Most people with these early changes (referred to as "age-related maculopathy") have good vision.

"AMD Risk Factor" encompasses one or more biomarker whose level is changed in subjects having age-related macular degeneration (AMD) or is predisposed to developing age-related macular degeneration (AMD), or at risk of age-related macular degeneration (AMD).

"Risk" in the context of the present invention, relates to the probability that an event will occur over a specific time period, as in the conversion to age-related macular degeneration (AMD), and can mean a subject's "absolute" risk or "relative" risk. Absolute risk can be measured with reference to either actual observation post-measurement for the relevant time cohort, or with reference to index values developed from statistically valid historical cohorts that have been followed for the relevant time period. Relative risk refers to the ratio of absolute risks of a subject compared either to the absolute risks of low risk cohorts or an average population risk, which can vary by how clinical risk factors are assessed. Odds ratios, the proportion of positive events to negative events for a given test result, are also commonly used (odds are according to the formula p/(l-p) where p is the probability of event and (1- p) is the probability of no event) to no- conversion. Alternative continuous measures which may be assessed in the context of the present invention include time to age-related macular degeneration (AMD) conversion and therapeutic age-related macular degeneration (AMD) conversion risk reduction ratios.

"Risk evaluation," or "evaluation of risk" in the context of the present invention encompasses making a prediction of the probability, odds, or likelihood that an event or disease state may occur, the rate of occurrence of the event or conversion from one disease state to another, i.e., from a normal condition to age-related macular degeneration (AMD) condition. Risk evaluation can also comprise prediction of future clinical parameters, traditional laboratory risk factor values, or other indices of age-related macular degeneration (AMD), such as metamorphosia and:or central vision loss, macular drusen, irregular proliferation and atrophy of the RPE, geographic atrophy, choroidal neovascularization, retinal angiomatosis proliferation... The methods of the present invention may be used to make continuous or categorical measurements of the risk of conversion to age-related macular degeneration (AMD), thus diagnosing and defining the risk spectrum of a category of subjects defined as being at risk for age-related macular degeneration (AMD). In the categorical scenario, the invention can be used to discriminate between normal and other subject cohorts at higher risk for age-related macular degeneration (AMD). In other embodiments, the present invention may be used so as to discriminate those having early late stages of age-related macular degeneration (named ARM for age related maculopathy) or late stages of age-related macular degeneration (AMD) from normal.

A "sample" in the context of the present invention is a biological sample isolated from a subject and can include, by way of example and not limitation, bodily fluids and/or tissue extracts such as homogenates or solubilized tissue obtained from a subject. Tissue extracts are obtained routinely from tissue biopsy and autopsy material. Bodily fluids useful in the present invention include blood, urine, saliva or any other bodily secretion or derivative thereof. As used herein "blood" includes whole blood, plasma, serum, circulating epithelial cells, constituents, or any derivative of blood.

A "subject" in the context of the present invention is preferably a mammal. The mammal can be a human, non-human primate, mouse, rat, dog, cat, horse, or cow, but are not limited to these examples. Mammals other than humans can be advantageously used as subjects that represent animal models of age-related macular degeneration (AMD) or age-related macular degeneration (AMD). A subject can be male or female. A subject can also be one who has not been previously diagnosed as having age-related macular degeneration (AMD). For example, a subject can be one who exhibits one or more risk factors for age-related macular degeneration (AMD), or a subject who does not exhibit age-related macular degeneration (AMD) risk factors, or a subject who is asymptomatic for age-related macular degeneration (AMD). A subject can also be one who is at risk of developing age-related macular degeneration (AMD).

As used herein, the term "SCARB1 gene" denotes the SCARB1 gene of any species, especially human, but also other mammals or vertebrates to which the methods of the invention can apply. The human SCARB1 gene is located at 12q24.31 locus. The SCARB1 gene encodes for SRB1 a multiligand cells surface receptor known to mediate selective cholesterol uptake and cholesterol efflux.

The terms "mutant" and "mutation" mean any detectable change in genetic material, e.g. DNA, RNA, cDNA, or any process, mechanism, or result of such a change. This includes gene mutations, in which the structure (e.g. DNA sequence) of a gene is altered, any gene or DNA arising from any mutation process, and any expression product (e.g. protein or enzyme) expressed by a modified gene or DNA sequence. Generally a mutation is identified in a subject by comparing the sequence of a nucleic acid or polypeptide expressed by said subject with the corresponding nucleic acid or polypeptide expressed in a control population. A mutation in the genetic material may also be "silent", i.e. the mutation does not result in an alteration of the amino acid sequence of the expression product.

The term "Allele" has the meaning which is commonly known in the art, that is, an alternative form of a gene (one member of a pair) that is located at a specific position on a specific chromosome which, when translated result in functional or dysfunctional (including non-existant) gene products.

The term "polymorphism" or "allelic variant" means a mutation in the normal sequence of a gene, Allelic variants can be found in the exons, introns, or the coding region of the gene, or in the sequences that control expression of the gene. Complete gene sequencing often identifies numerous allelic variants (sometimes hundreds) for a given gene. The significance of allelic variants is often unclear until further study of the genotype and corresponding phenotype occurs in a sufficiently large population.

The term "Single nucleotide polymorphism" or "SNP" means a single nucleotide variation in a genetic sequence that occurs at appreciable frequency in the population. There are millions of SNPs in the human genome. Most commonly, these variations are found in the DNA between genes. When SNPs occur within a gene or in a regulatory region near a gene, they may play a more direct role in disease by affecting the gene's function. All the SNPs pertaining to the invention (e.g. rs5888) are known per se and sequences of them are publicly available from the data base http://www.ncbi.nlm.nih.gov/SNP/ or http://hapmap.ncbi.nlm.nih.gov/. The term "linkage disequilibrium" (LD) refers to a population association among alleles at two or more loci. It is a measure of co-segregation of alleles in a population. Linkage disequilibrium or allelic association is the preferential association of a particular allele or genetic marker with a specific allele, or genetic marker at a nearby chromosomal location more frequently than expected by chance for any particular allele frequency in the population.

A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength (see Sambrook et al., 1989).

The conditions of temperature and ionic strength determine the "stringency" of the hybridization. For preliminary screening for homologous nucleic acids, low stringency hybridization conditions, corresponding to a Tm (melting temperature) of 55°C, can be used, e.g., 5x SSC, 0.1 % SDS, 0.25 % milk, and no formamide ; or 30 % formamide, 5x SSC, 0.5 % SDS). Moderate stringency hybridization conditions correspond to a higher Tm, e.g., 40 % formamide, with 5x or 6x SCC. High stringency hybridization conditions correspond to the highest Tm, e.g., 50 % formamide, 5x or 6x SCC. SCC is a 0.15 M NaCl, 0.015 M Na-citrate. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook et al., 1989, 9.50-9.51). For hybridization with shorter nucleic acids, i.e., oligonucleotides, the position of mismatches becomes more important, and the length of the oligonucleotide determines its specificity (see Sambrook et al., 1989 11.7-11.8). A minimum length for a hybridizable nucleic acid is at least about 10 nucleotides, preferably at least about 15 nucleotides, and more preferably the length is at least about 20 nucleotides.

In a specific embodiment, the term "standard hybridization conditions" refers to a Tm of 55°C, and utilizes conditions as set forth above. In a preferred embodiment, the Tm is 60°C. In a more preferred embodiment, the Tm is 65°C. In a specific embodiment, "high stringency" refers to hybridization and/or washing conditions at 68°C in 0.2 X SSC, at 42°C in 50 % formamide, 4 X SSC, or under conditions that afford levels of hybridization equivalent to those observed under either of these two conditions.

As used herein, an amplification primer is an oligonucleotide for amplification of a target sequence by extension of the oligonucleotide after hybridization to the target sequence or by ligation of multiple oligonucleotides which are adjacent when hybridized to the target sequence. At least a portion of the amplification primer hybridizes to the target. This portion is referred to as the target binding sequence and it determines the target-specificity of the primer. In addition to the target binding sequence, certain amplification methods require specialized non-target binding sequences in the amplification primer. These specialized sequences are necessary for the amplification reaction to proceed and typically serve to append the specialized sequence to the target. For example, the amplification primers used in Strand Displacement Amplification (SDA) include a restriction endonuclease recognition site 5' to the target binding sequence (US Patent No. 5,455,166 and US Patent No. 5,270,184). Nucleic Acid Based Amplification (NASBA), self-sustaining sequence replication (3SR) and transcription based amplification primers require an RNA polymerase promoter linked to the target binding sequence of the primer. Linking such specialized sequences to a target binding sequence for use in a selected amplification reaction is routine in the art. In contrast, amplification methods such as PCR which do not require specialized sequences at the ends of the target, generally employ amplification primers consisting of only target binding sequence.

As used herein, the terms "primer" and "probe" refer to the function of the oligonucleotide. A primer is typically extended by polymerase or ligation following hybridization to the target but a probe typically is not. A hybridized oligonucleotide may function as a probe if it is used to capture or detect a target sequence, and the same oligonucleotide may function as a primer when it is employed as a target binding sequence in an amplification primer. It will therefore be appreciated that any of the target binding sequences disclosed herein for amplification, detection or quantisation of SCARB1 may be used either as hybridization probes or as target binding sequences in primers for detection or amplification, optionally linked to a specialized sequence required by the selected amplification reaction or to facilitate detection.

In the context of the invention, the term "treating" or "treatment", as used herein, means reversing, alleviating, inhibiting the progress of, or preventing the disorder or condition to which such term applies, or one or more symptoms of such disorder or condition. A "therapeutically effective amount" is intended for a minimal amount of active agent (e.g., SCARB1 encoding polynucleotide) which is necessary to impart therapeutic benefit to a subject. For example, a "therapeutically effective amount" to a mammal is such an amount which induces, ameliorates or otherwise causes an improvement in the pathological symptoms, disease progression or physiological conditions associated with or resistance to succumbing to a disorder.

### Diagnostic methods of the invention:

The purpose of the inventors was to assess candidate genes and polymorphisms involved in the lipid pathway among individuals harboring non-risk alleles for CFH and ARMS2. The SCARB1 gene which encodes SRB1, a multiligand cell surface receptor, is known to mediate selective cholesterol uptake, and cholesterol efflux, and was regarded as an attractive candidate gene. Furthermore, recent studies have shown that SCARB1 is also involved in uptake of vitamin E and lutein giving further support to a possible role of SCARB1 in AMD. In fact, the xanthophyll, lutein, is recovered at high concentration in the human macula lutea and has been associated with the risk of AMD, and vitamin E, the main lipophilic antioxidant, is suspected to prevent oxidation of polyunsaturated fatty acids recovered at high concentrations in the human retina. Several studies have suggested that SCARB1 genotypes for the rs5888 synonymous SNP may play a role in cholesterol homeostasis and is associated with age-related macular degenerations. Herein, the inventors report an association between the SCARB1 rs5888 SNP and AMD in a subgroup of French and North American AMD subjects in a case-control study.

Therefore, the present invention relates to a method for diagnosing or predicting age related maculopathy or age-related macular degeneration, or a risk of age related maculopathy or age-related macular degeneration, in a subject, said method comprising detecting in a sample obtained from said subject at least one polymorphism in the SCARB1 gene, wherein the presence of said polymorphism is indicative of non age related maculopathy or age-related macular degeneration or of a risk of age related maculopathy or age-related macular degeneration.

A further object of the invention is a method of identifying a subject having or at risk of having or developing age related maculopathy or age-related macular degeneration, comprising detecting, in a sample obtained from said subject, the single nucleotide polymorphism rs5888 in the SCARB1 gene wherein the presence of the minor allele (T) of rs5888 indicates an increased risk of having or being at risk of having or developing age related maculopathy or age-related macular degeneration.

A further object of the invention is a method of identifying a subject having or at risk of having or developing age related maculopathy or age-related macular degeneration, comprising detecting, in a sample obtained from said subject, at least one polymorphism in linkage disequilibrium with rs5888 nucleotide polymorphism (SNP) in the SCARB1 gene wherein the presence said polymorphism indicates an increased risk of having or being at risk of having or developing age related maculopathy or age-related macular degeneration.

In one embodiment of the invention, the subject having or being at risk of having or developing age-related macular degeneration (AMD) may be a substantially healthy subject, which means that the subject has not been previously diagnosed or identified as having or suffering from age-related macular degeneration (AMD).

In another embodiment, said subject may also be one that is asymptomatic for the age-related macular degeneration (AMD). As used herein, an "asymptomatic" subject refers to a subject that does not exhibit the traditional symptoms of age-related macular degeneration (AMD).

In another embodiment of the invention, said subject may be one that is at risk of having or developing age-related macular degeneration (AMD), as defined by clinical indicia such as for example: age, gender, family history of age-related macular degeneration (AMD), smoking, high body mass index, high HDL lipids, precursors of AMD on fundus examination.

In one embodiment of the invention, the subject is a mammal, preferably a human.

In another embodiment of the invention, the sample obtained from the subject comprises bodily fluids (such as blood, saliva or any other bodily secretion or derivative thereof), and/or tissue extracts such as homogenates or solubilized tissue obtained from the subject. In a preferred embodiment, the sample to be tested is blood.

According to the invention, the sample comprises SCARB1 nucleic acid, wherein the SCARB1 nucleic acid may be genomic DNA, heterogenous nuclear RNA (hnRNA, also referred as incompletely processed single strand of ribonucleic acid) and/or cDNA.

According to the invention, the determination of the presence or absence of said polymorphism may be determined by nucleic acid sequencing, PCR analysis or any genotyping method known in the art. Examples of such methods include, but are not limited to, chemical assays such as allele specific hybridation, primer extension, allele specific oligonucleotide ligation, sequencing, enzymatic cleavage, flap endonuclease discrimination; and detection methods such as fluorescence, chemiluminescence, and mass spectrometry.

For example, the presence or absence of said polymorphism may be detected in a RNA or DNA sample, preferably after amplification. For instance, the isolated RNA may be subjected to coupled reverse transcription and amplification, such as reverse transcription and amplification by polymerase chain reaction (RT-PCR), using specific oligonucleotide primers that are specific for the polymorphism or that enable amplification of a region containing the polymorphism. According to a first alternative, conditions for primer annealing may be chosen to ensure specific reverse transcription (where appropriate) and amplification; so that the appearance of an amplification product be a diagnostic of the presence of the polymorphism according to the invention. Otherwise, RNA may be reverse-transcribed and amplified, or DNA may be amplified, after which a mutated site may be detected in the amplified sequence by hybridization with a suitable probe or by direct sequencing, or any other appropriate method known in the art. For instance, a cDNA obtained from RNA may be cloned and sequenced to identify the polymorphism.

Actually numerous strategies for genotype analysis are available. Briefly, the nucleic acid molecule may be tested for the presence or absence of a restriction site. When a base polymorphism creates or abolishes the recognition site of a restriction enzyme, this allows a simple direct PCR test for the polymorphism. Further strategies include, but are not limited to, direct sequencing, restriction fragment length polymorphism (RFLP) analysis; hybridization with allele-specific oligonucleotides (ASO) that are short synthetic probes which hybridize only to a perfectly matched sequence under suitably stringent hybridization conditions; allele-specific PCR; PCR using mutagenic primers; ligase-PCR, HOT cleavage; denaturing gradient gel electrophoresis (DGGE), temperature denaturing gradient gel electrophoresis (TGGE), single-stranded conformational polymorphism (SSCP) and denaturing high performance liquid chromatography. Direct sequencing may be accomplished by any method, including without limitation chemical sequencing, using the Maxam-Gilbert method ; by enzymatic sequencing, using the Sanger method ; mass spectrometry sequencing ; sequencing using a chip-based technology; and real-time quantitative PCR. Preferably, DNA from a subject is first subjected to amplification by polymerase chain reaction (PCR) using specific amplification primers. However several other methods are available, allowing DNA to be studied independently of PCR, such as the rolling circle amplification (RCA), the InvaderTMassay, or oligonucleotide ligation assay (OLA). OLA may be used for revealing base polymorphisms. According to this method, two oligonucleotides are constructed that hybridize to adjacent sequences in the target nucleic acid, with the join sited at the position of the polymorphism. DNA ligase will covalently join the two oligonucleotides only if they are perfectly hybridized.

Therefore, useful nucleic acid molecules, in particular oligonucleotide probes or primers, according to the present invention include those which specifically hybridize the regions where the polymorphisms are located.

Oligonucleotide probes or primers may contain at least 10, 15, 20 or 30 nucleotides. Their length may be shorter than 400, 300, 200 or 100 nucleotides.

According to a further embodiment said polymorphism in the SCARB1 gene may be detected at the protein level.

Said polymorphism may be detected according to any appropriate method known in the art. In particular a sample obtained from a subject may be contacted with antibodies specific of the mutated form of SRB1 which results from the polymorphism according to the invention, i.e. antibodies that are capable of distinguishing between a mutated form of SRB1 and the wild-type protein (or any other protein), to determine the presence or absence of a SRB1 specified by the antibody.

Antibodies that specifically recognize a mutated SRB1 also make part of the invention. The antibodies are specific of mutated SRB that is to say they do not cross-react with the wild-type SRB1.

The antibodies of the present invention may be monoclonal or polyclonal antibodies, single chain or double chain, chimeric antibodies, humanized antibodies, or portions of an immunoglobulin molecule, including those portions known in the art as antigen binding fragments Fab, Fab', F(ab')2 and F(v). They can also be immunoconjugated, e.g. with a toxin, or labelled antibodies.

Whereas polyclonal antibodies may be used, monoclonal antibodies are preferred for they are more reproducible in the long run.

Procedures for raising "polyclonal antibodies" are also well known. Polyclonal antibodies can be obtained from serum of an animal immunized against the appropriate antigen, which may be produced by genetic engineering for example according to standard methods well-known by one skilled in the art. Typically, such antibodies can be raised by administering mutated SRB1 subcutaneously to New Zealand white rabbits which have first been bled to obtain pre-immune serum. The antigens can be injected at a total volume of 100 µl per site at six different sites. Each injected material may contain adjuvants with or without pulverized acrylamide gel containing the protein or polypeptide after SDS-polyacrylamide gel electrophoresis. The rabbits are then bled two weeks after the first injection and periodically boosted with the same antigen three times every six weeks. A sample of serum is then collected 10 days after each boost. Polyclonal antibodies are then recovered from the serum by affinity chromatography using the corresponding antigen to capture the antibody..

A "monoclonal antibody" in its various grammatical forms refers to a population of antibody molecules that contains only one species of antibody combining site capable of immunoreacting with a particular epitope. A monoclonal antibody thus typically displays a single binding affinity for any epitope with which it immunoreacts. A monoclonal antibody may therefore contain an antibody molecule having a plurality of antibody combining sites, each immunospecific for a different epitope, e.g. a bispecific monoclonal antibody. Although historically a monoclonal antibody was produced by immortalization of a clonally pure immunoglobulin secreting cell line, a monoclonally pure population of antibody molecules can also be prepared by the methods of the present invention.

Laboratory methods for preparing monoclonal antibodies are well known in the art. Monoclonal antibodies (mAbs) may be prepared by immunizing purified mutated SRB1 into a mammal, e.g. a mouse, rat, human and the like mammals. The antibody-producing cells in the immunized mammal are isolated and fused with myeloma or heteromyeloma cells to produce hybrid cells (hybridoma). The hybridoma cells producing the monoclonal antibodies are utilized as a source of the desired monoclonal antibody.

While mAbs can be produced by hybridoma culture the invention is not to be so limited. Also contemplated is the use of mAbs produced by an expressing nucleic acid cloned from a hybridoma of this invention. That is, the nucleic acid expressing the molecules secreted by a hybridoma of this invention can be transferred into another cell line to produce a transformant. The transformant is genotypically distinct from the original hybridoma but is also capable of producing antibody molecules of this invention, including immunologically active fragments of whole antibody molecules, corresponding to those secreted by the hybridoma. See, for example, U.S. Pat. No. 4,642,334 to Reading; PCT Publication No.; European Patent Publications No. 0239400 to Winter et al. and No. 0125023 to Cabilly et al.

Antibody generation techniques not involving immunisation are also contemplated such as for example using phage display technology to examine naive libraries (from non-immunised animals).

Antibodies raised against mutated SRB1 may be cross reactive with wild-type SRB1. Accordingly a selection of antibodies specific for mutated SRB1 is required. This may be achieved by depleting the pool of antibodies from those that are reactive with the wild-type SRB1, for instance by submitting the raised antibodies to an affinity chromatography against wild-type SRB1.

Alternatively, binding agents other than antibodies may be used for the purpose of the invention. These may be for instance aptamers, which are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library The random sequence library is obtainable by combinatorial chemical synthesis of DNA. In this library, each member is a linear oligomer, eventually chemically modified, of a unique sequence.. Peptide aptamers consists of a conformationally constrained antibody variable region displayed by a platform protein, such as E. coli Thioredoxin A that are selected from combinatorial libraries by two hybrid methods.

Probe, primers, aptamers or antibodies of the invention may be labelled with a detectable molecule or substance, such as a fluorescent molecule, a radioactive molecule or any others labels known in the art. Labels are known in the art that generally provide (either directly or indirectly) a signal.

The term "labelled", with regard to the probe, primers, aptamers or antibodies of the invention, is intended to encompass direct labelling of the the probe, primers, aptamers or antibodies of the invention by coupling (i.e., physically linking) a detectable substance to the the probe, primers, aptamers or antibodies of the invention, as well as indirect labeling of the probe, primers, aptamers or antibodies of the invention by reactivity with another reagent that is directly labeled. Other examples of detectable substances include but are not limited to radioactive agents or a fluorophore (e.g. fluorescein isothiocyanate (FITC) or phycoerythrin (PE) or Indocyaninc (Cy5)). Examples of indirect labeling include detection of a primary antibody using a fluorescently labeled secondary antibody and end-labeling of a DNA probe with biotin such that it can be detected with fluorescently labeled streptavidin. An antibody or aptamer of the invention may be labelled with a radioactive molecule by any method known in the art. For example radioactive molecules include but are not limited radioactive atom for scintigraphic studies such as I123, I124, In111, Re186, Re188.

Therefore, the present invention relates to a method for predicting the responsiveness of a subject affected with age-related maculopathy or age-related macular degeneration to an antioxydative therapy, said method comprising detecting in a sample obtained from said subject at least one polymorphism in the SCARB1 gene, wherein the presence of said polymorphism indicates that said patient is a non responder to said therapy.

A further object of the invention is a method for predicting the responsiveness of a subject affected with age-related maculopathy or age-related macular degeneration to an antioxydative therapy comprising detecting, in a sample obtained from said subject, the single nucleotide polymorphism rs5888 in the SCARB1 gene wherein the presence of the minor allele (T) of rs5888 indicates that said patient is a non responder to said therapy.

A further object of the invention is a method for predicting the responsiveness of a subject affected with age-related maculopathy or age-related macular degeneration to an antioxydative therapy, comprising detecting, in a sample obtained from said subject, at least one polymorphism in linkage disequilibrium with rs5888 nucleotide polymorphism (SNP) in the SCARB1 gene wherein the presence said polymorphism indicates that said patient is a non responder to said therapy.

As used herein, the term "non responder" or "non responsive subject", or group of subjects, to an antioxydative therapy, refers to a subject, or group of subjects, who does not show or will not show a clinically significant relief in the age-related macular degeneration when administered with said antioxydative therapy.

In one embodiment, the antioxydative therapy may consist in administering with an amount of antioxydants selected from the group consisting of vitamin C, vitamin E, betacaroten, genistein, lutein, zeaxanthin and mineral zincs.

### Kits of the invention:

A further object of the invention is a kit for performing the methods of the invention, comprising at least one primer and/or at least one probe for amplification of a sequence comprising the polymorphisms of the invention and instructions for use.

In one embodiment of the invention, the primer or probe may be labelled with a suitable marker. In another embodiment of the invention, the primer or probe may be coated on an array.

Alternatively, the kit of the invention may comprise a labelled compound or agent capable of detecting the mutated polypeptide of the invention (e.g., an antibody or aptamers as described above which binds the polypeptide). For example, the kit may comprise (1) a first antibody (e.g., attached to a solid support) which binds to a polypeptide comprising a mutation of the invention; and, optionally, (2) a second, different antibody which binds to either the polypeptide or the first antibody and is conjugated to a detectable agent.

The kit can also comprise, e.g., a buffering agent, a preservative, or a protein stabilizing agent. The kit can also comprise components necessary for detecting the detectable agent (e.g., an enzyme or a substrate). Each component of the kit is usually enclosed within an individual container and all of the various containers are within a single package along with instructions for observing whether the tested subject is suffering from or is at risk of developing an age-related macular degeneration.

The kit can include clinical data such as phenotype of the macula and questionnaire of the patient.

### Screening methods of the invention:

The present invention also provides novel targets and methods for the screening of drug candidates or leads. Such drug candidates or leads are useful for developing a treatment for age-related macular degeneration. The methods include binding assays and/or functional assays, and may be performed in vitro, in cell systems, in animals, etc. Functional assays comprise, but are not limited to, metabolism of cholesterol, lutein and vitamin E assays. The in vitro assays, cell-based assays and animal-based assays involve a SRB1 protein.

The invention relates to methods for screening of compounds that modulate the SRB1 activity. Such compounds, for example, can increase or decrease affinity and/or rate of binding of the SRB1 protein to the substrate (e.g. cholesterol, lutein or vitamin E), compete with substrate for binding to the SRB1 protein, or displace substrate bound to the SRB1 protein. Preferably, the invention concerns methods for screening of compounds that increase or restore the natural SRB1 activity. By "natural" SRB1 activity is intended the activity of the wild-type SRB1 protein. Furthermore, the invention concerns methods for screening of compounds that inhibit the activity of the altered SRB1 comprising an alteration changing the substrate specificity and, thereby generating new substrates.

Therefore, the present invention concerns a method of selecting biologically active compounds, said method comprising contacting a test compound with an altered SCARB1 gene or an altered SRB1 protein or fragment thereof of at least 15 consecutive residues comprising an alteration, wherein the alteration reduces, modifies, or abolishes the activity of SRB1, and determining the ability of said test compound to modulate the expression and/or activity of said gene or protein or fragment.

A particular object of this invention resides in a method of selecting biologically active compounds, said method comprising contacting in vitro a test compound with a SCARB1 gene or SRB1 polypeptide, preferably a SCARB1 gene or a SRB1 polypeptide, or a fragment thereof of at least 15 consecutive residues, comprising at least one polymorphism according to the invention according to the present invention, and determining the ability of said test compound to bind said SCARB1 gene or SRB1 polypeptide. Binding to said gene or polypeptide provides an indication as to the ability of the compound to modulate the activity of said target, and thus to affect a pathway leading to age-related macular degeneration in a subject. In a preferred embodiment, the method comprises contacting in vitro a test compound with a SRB1 polypeptide or a fragment thereof, preferably a SRB1 polypeptide or a fragment thereof comprising at least one polymorphism according to the invention according to the present invention, and determining the ability of said test compound to bind said SRB1 polypeptide or fragment. The fragment preferably comprises a substrate-binding site of the SRB1 polypeptide.

A particular object of this invention resides in a method of selecting compounds active for the treatment of age-related macular degeneration, said method comprising contacting in vitro a test compound with a SRB1 polypeptide or a fragment thereof of at least 15 consecutive residues, preferably a SRB1 polypeptide or a fragment thereof comprising at least one polymorphism according to the invention according to the present invention, and determining the ability of said test compound to bind said SRB1 polypeptide or fragment thereof. The SRB1 polypeptide or fragment thereof may be used in essentially pure form, in suspension, or immobilized on a support.

In a further particular embodiment, the method comprises contacting a recombinant host cell expressing SRB1 polypeptide, preferably a SRB1 polypeptide comprising at least one polymorphism according to the invention according to the present invention, with a test compound, and determining the ability of said test compound to bind said SRB1 polypeptide and/or to modulate the activity of SRB1 polypeptide.

The determination of binding may be performed by various techniques, such as by labelling of the test compound, by competition with a labelled reference ligand, two-hybrid Screening Assay, etc. Modulation of activity includes, without limitation, the inhibition or activation of the cholesterol uptake and efflux

A further object of this invention resides in a method of selecting biologically active compounds, said method comprising contacting in vitro a test compound with a SRB1 polypeptide, preferably a SRB1 polypeptide comprising at least one polymorphism according to the invention according to the present invention, and determining the ability of said test compound to modulate the activity of said SRB1 polypeptide.

A further object of this invention resides in a method of selecting biologically active compounds, said method comprising contacting in vitro a test compound with a SCARB1 gene, preferably a SCARB1 gene comprising at least one polymorphism according to the invention according to the present invention, and determining the ability of said test compound to modulate the expression of said SCARAB1 gene.

The invention also concerns methods of selecting biologically active compounds using a non-human transgenic animals expressing a SRB1 protein, preferably a SRB1 protein comprising at least one polymorphism according to the invention according to the present invention. Optionally, said non-human transgenic animals can be homozygote or heterozygote for the altered SCARB1 gene. Said methods comprise (i) administrating a test compound to said non-human transgenic animal, and (ii) determining the ability of said test compound to modulate the SCARB1 activity. Said SCARB1 activity can be assessed by evaluating the cholesterol, vitamin E and lutein metabolism.

The above screening assays may be performed in any suitable device, such as plates, tubes, dishes, flasks, etc. Typically, the assay is performed in multi-wells plates. Several test compounds can be assayed in parallel.

Furthermore, the test compound may be of various origin, nature and composition. It may be any organic or inorganic substance, such as a lipid, peptide, polypeptide, nucleic acid, small molecule, etc., in isolated or in mixture with other substances. The compounds may be all or part of a combinatorial library of products, for instance. The test compounds can be an antisense or an RNAi. The test compounds can be competitive or suicide substrates. By "suicide substate" is intended a compounds that, after binding SRB1 protein, the reactive group forms an irreversible bond with SRB1 rendering it inactive.

### Therapeutic methods of the invention:

In a further object, the invention relates to use, methods and pharmaceutical compositions for treating or preventing age-related macular degeneration. Gene therapy is a particularly convenient way to treat age-related macular degeneration as it enables the provision of a constant supply of polypeptide or correction of the defective gene, for example as discussed below.

Gene therapy may be carried out by means of supplementation of cells lacking a functional SRB1 polypeptide with a wild type SCARB1 gene product. Production of a suitable gene product may be achieved using recombinant techniques. For example, a suitable vector may be inserted into a host cell and expressed in that cell.

Thus the invention further relates to a method for treating or preventing age-related macular degeneration which comprises the step of administering a subject in need thereof with a SCARB1 polynucleotide, i.e. a nucleic acid sequence that encodes a wild-type SCARB1, so that SCARB1 is expressed in vivo by the cells of the subject that have been transfected with said polynucleotide. Accordingly, said method leads to an overexpression of wild-type SCARB1 which compensates expression of defective mutated SCARB1.

The invention also relates to the use of a SCARB1 polynucleotide for the manufacture of medicament intended for the treatment of an age-related macular degeneration.

Said SCARB1 polynucleotide is administered in a therapeutically effective amount.

Preferably the SCARB1 polynucleotide sequence according to the invention is associated with elements that enable for regulation of its expression, such as a promoter sequence.

Such a nucleic acid may be in the form of a vector. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors, expression vectors, are capable of directing the expression of genes to which they are operably linked. In general, expression vectors of utility in recombinant DNA techniques are often in the form of plasmids (vectors). However, the invention is intended to include such other forms of expression vectors, such as viral vectors (e.g., replication defective retroviruses, adenoviruses and adeno-associated viruses (AAV)), which serve equivalent functions.

In a preferred embodiment, the expression vector is an AAV vector. Adeno-associated viral (AAV) vectors have become powerful gene delivery tools for the treatment of retinal degeneration. AAV vectors possess a number of features that render them ideally suited for retinal gene therapy, including a lack of pathogenicity, minimal immunogenicity, and the ability to transduce postmitotic cells in a stable and efficient manner. In the sheltered environment of the retina, AAV vectors are able to maintain high levels of transgene expression in the retinal pigmented epithelium (RPE), photoreceptors, or ganglion cells for long periods of time after a single treatment. Each cell type can be specifically targeted by choosing the appropriate combination of AAV serotype, promoter, and intraocular injection site.

The SCARB1 polynucleotide may be introduced into a target cell by means of any procedure known for the delivery of nucleic acids to the nucleus of cells, ex vivo, on cells in culture or removed from an animal or a subject, or in vivo.

Ex vivo introduction may be performed by any standard method well known by one skilled in the art, e.g. transfection, electroporation, iontophoresis, microinjection, transduction, cell fusion, DEAE dextran, calcium phosphate precipitation, or use of a gene gun.

The SCARB1 polynucleotide can also be introduced ex vivo or in vivo by lipofection. In certain embodiments, the use of liposomes and/or nanoparticles is contemplated for the introduction of the donor nucleic acid targeting system into host cells.

Nanocapsules can generally entrap compounds in a stable and reproducible way. To avoid side effects due to intracellular polymeric overloading, such ultrafine particles (sized around 0.1 µm) should be designed using polymers able to be degraded in vivo. Biodegradable polyalkyl-cyanoacrylate nanoparticles that meet these requirements are contemplated for use in the present invention, and such particles may be are easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm. Sonication of MLVs results in the formation of small unilamellar vesicles (SUVs) with diameters in the range of 200 to 500 Å, containing an aqueous solution in the core.

Synthetic cationic lipids designed to limit the difficulties and dangers encountered with liposome mediated transfection can be used to prepare liposomes for in vivo transfection of a gene encoding a marker. The use of cationic lipids may promote encapsulation of negatively charged nucleic acids, and also promote fusion with negatively charged cell membranes.

Alternatively, one of the simplest and the safest way to deliver SCARB1 polynucleotide across cell membranes in vivo may involve the direct application of high concentration free or naked polynucleotides (typically mRNA or DNA). By "naked DNA (or RNA)" is meant a DNA (RNA) molecule which has not been previously complexed with other chemical moieties. Naked DNA uptake by animal cells may be increased by administering the cells simultaneously with excipients and the nucleic acid. Such excipients are reagents that enhance or increase penetration of the DNA across cellular membranes and thus delivery to the cells delivery of the therapeutic agent. Various excipients have been described in the art, such as surfactants, e.g. a surfactant selected form the group consisting of Triton X-100, sodium dodecyl sulfate, Tween 20, and Tween 80; bacterial toxins, for instance streptolysin O, cholera toxin, and recombinant modified labile toxin of E coli; and polysaccharides, such as glucose, sucrose, fructose, or maltose, for instance, which act by disrupting the osmotic pressure in the vicinity of the cell membrane. Other methods have been described to enhance delivery of free polynucleotides, such as blocking of polynucleotide inactivation via endo- or exonucleolytic cleavage by both extra- and intracellular nucleases.

Alternatively, the invention also provides a method for treating or preventing age-related macular degeneration which comprises the step of administering a subject in need thereof with a wild-type SRB1 polypeptide.

Knowing the amino acid sequence of the desired sequence, one skilled in the art can readily produce said polypeptides, by standard techniques for production of polypeptides. For instance, they can be synthesized using well-known solid phase method, preferably using a commercially available peptide synthesis apparatus (such as that made by Applied Biosystems, Foster City, California) and following the manufacturer's instructions.

Alternatively, the polypeptides of the invention can be synthesized by recombinant DNA techniques as is now well-known in the art. For example, these fragments can be obtained as DNA expression products after incorporation of DNA sequences encoding the desired (poly)peptide into expression vectors and introduction of such vectors into suitable eukaryotic or prokaryotic hosts that will express the desired polypeptide, from which they can be later isolated using well-known techniques.

Polypeptides of the invention can be use in an isolated (e.g., purified) form or contained in a vector, such as a membrane or lipid vesicle (e.g. a liposome).

Delivery of SCARB1 gene, antibodies or protein can also be performed directly in the eye by intra vitreal injection.

### Transgenic non-human animal models of age-related macular degeneration:

The present invention further relates to an in vivo model of age-related macular degeneration wherein said animal is a transgenic non-human animal which is SCARB1-deficient.

By transgenic non-human animal which is SCARB1-deficient, it is meant a homozygous animal (i.e., the two alleles of SCARB1 are defective).

Typically suitable transgenic non-human animals are laboratory animals such as rodents, rats, mice, monkeys, dogs, rabbits, guinea pigs, goats, sheep, pigs and cattle. In one embodiment the transgenic non-human animal of the present invention is a rodent. In a more preferred embodiment the transgenic animal is a mouse.

Typically the transgenic animals according to the invention are generated using General Methods Standard molecular biology techniques known in the art (see for example US Patent 6740793, US2006/0101533).

The invention provides a means for identification of agents that interfere, delay or inhibit processes involved in diseases or conditions involving a mutated SCARB1 gene, such as age-related macular degeneration. Such agents would be of significant clinical importance for treatment of age-related macular degeneration. The provision of the animal model according to the present invention can greatly shorten the time required for screening for such agents. Thus the present invention relates to a method for identifying a compound useful for treating, preventing or inhibiting age-related macular degeneration, comprising the step of contacting a candidate compound with a transgenic animal according to the invention.

The invention will be further illustrated by the following figures and examples.

### EXAMPLE

### Methods:

### French Populations

### Patients

Written informed consent was obtained, as required by the French bioethical legislation and local ethic committee (CCPPRB Henri Mondor), in agreement with the Declaration of Helsinki for research involving human subjects.

A total of 1241 French AMD patients were recruited in 4 French Ophthalmologic Centres, at the Ophthalmology Eye Clinic of Créteil in collaboration with the Pellegrin Hospital, the Quinze-Vingts Hospital and the Centre of Imaging and Laser of Paris, between November 2005 and July 2007. Inclusion criteria of the AMD patients were (1) women or men aged 55 or older, and (2) with exudative AMD, atrophic AMD or with early or intermediate AMD (also called Aged-Related Maculopathy) in at least one eye. Exclusion criteria were presence of other retinal disease (e.g. diabetic retinopathy, high myopia, or macular dystrophies). Patients underwent a complete ophthalmologic examination including best corrected visual acuity measurement, fundus examination, and retinal photographs. Fluorescein angiography (Topcon 50IA camera, Tokyo, Japan)- and if needed indocyanine green angiography (HRA, Heidelberg, Germany)- and Optical Coherence Tomography (Carl Zeiss Meditec, Inc) were performed. A questionnaire about medical history and smoking was completed.

### Controls

A total of 297 French women or men over 55 years with a normal fundus examination and a normal aspect of fundus photography were also recruited at the Ophtalmology Eye Clinic of Créteil between 2002 and 2008. Information about their medical history including smoking, was obtained.

### Genotyping Methods

Genomic DNA was extracted from 10 mL blood leukocytes using the Illustra® kit according to the manufacturer protocol (GE Healthcare). The *SCARB1* rs5888 *CFH* Y402H and *ARMS2* rs10490924 SNPs were genoyped by quantitative PCR allelic discrimination using reagents and conditions from Custom Taqman SNP Genotyping Assays (Applera Corp., France), using ABI 7900HT (Applied Biosystems).

### North American Populations

### Patients

Subjects and methods of diagnosis and enrollment have been previously described. All patients had advanced age-related macular degeneration, either exudative or geographic atrophy, and diagnosis was based on ocular examination and fundus photography. They were all Caucasian and unrelated (Table 1).

### Controls

Caucasian individuals without AMD who were unrelated to the cases and to other controls were enrolled. Absence of AMD was based on ocular examination and grading of fundus photographs (Table 1). All cases and controls signed a written informed consent form.

### Genotyping Methods

DNA samples were evaluated for the rs5888 SNP using either the Affymetrix 6.0 platform as part of our genome-wide association study (under review) or the Sequenom platform.. Genotyping was performed at the Broad Institute in Cambridge, MA, USA.

### Statistical Analysis

Hardy-Weinberg assumption was assessed by the standard method comparing the observed numbers of subjects in different genotype categories with the expected number under Hardy-Weinberg equilibrium for the estimated allele frequency, and testing with a Pearson goodness-of-fit statistic with the χ² with 1 degree of freedom.

χ² test was used to compare categorical allelic and genotype distributions between cases and controls (table 1). General linear models were used to compare means between cases and controls. Logistic regression models were used to estimated odds ratio (OR) with 95% confidence interval (95%Cl) for AMD risk. OR's were adjusted for age, gender and smoking status. Significance levels were set at p<0.05. Analyses were performed with the SAS software release 9.01 (SAS Institute INC, Cary, NC).

Homogeneity between the 2 populations was tested by introduction of interaction terms with study center in the models (1rst test), by Breslow-day Test for homogeneity of the odds ratio (2nd test) and by I² (= % of heterogeneity). I² has been estimated by the software Review Manager 5. I² and Breslow-Day test have been made without adjustment.

### Results:

The French cohort consisted of 1241 cases (92% exudative AMD, 5.2% geographic atrophy, 2.8% early or intermediate AMD) and 297 controls. The mean ± SD age at AMD diagnosis was 78.8 ± 7.5 years. The North American cohort consisted of 1732 advanced AMD cases (72.4% exudative AMD, 27.6% geographic atrophy) and 1257 controls. All subjects were Caucasian. The mean ± SD age at AMD diagnosis was 80.3 ± 6.5 years (Table 1).

The genotype distributions of the rs1061170, rs10490924 and rs5888 SNPs within the *CFH, ARMS2* and *SCARB1* genes, respectively, are shown in Table 2. The genotypic distributions of the *CFH Y402H* and *ARMS2* SNPs were significantly different between cases and controls in both the French and North American series (p<0.0001 for both groups). For rs5888 genotypes, regardless of *CFH* or *ARMS2* genotypes, no significant association was found in the entire French population of AMD patients (Table 2). However, the distribution of the *SCARB1* rs5888 genotype was significantly different in the North American AMD population compared to controls (p<0.004): CT heterozygotes were at increased risk of AMD compared to CC subjects (adjusted OR_{CT vs cc}=1.4, 95%CI 1.0-1.8), TT did not significantly differ from CC (adjusted OR=1.2 CI95% 0.9-1.7).Similar results were obtained after pooling the French and the North American population : adjusted OR_{CT vs CC}=1.3, 95%CI: 1.0-1.7) and adjusted OR_{TT vs CC}=1.2, 95%CI 0.9-1.7. Odds Ratio were adjusted for non genetic confounders: age, gender and smoking status.

Characteristics of subjects carrying no risk alleles at the *CFH* and ARMS2 loci are shown in Table 3 (and Table S1). In the French population, genotypic distribution of the *SCARB1* polymorphism was significantly (p<0.01) different between cases and controls: CT heterozygotes compared to CC subjects were at increased risk of AMD (adjusted OR 3.5, 95%CI1.4-8.9). In the North American population, we observed a suggested increased risk of AMD associated with heterozygocity at the rs5888 (global test p< 0.09) : OR_{CT vs CC}=2.5; 95%CI 1.1-5.7. Similar results were obtained when pooling French and North American populations (global test, p < 0.002), in that CT individuals were at increased risk of AMD compared to CC genotypes: adjusted OR=2.9; 95%CI1.6-5.3 while TT subjects did not significantly differ from CC subjects (Table 4).

In exudative AMD subgroups (Cases: n=105, 55 French and 50 American ; controls: n=415, 77 French and 338 American), adjusted OR after pooling both populations for CT heterozygous individuals was: OR=3.6, 95%CI: 1.7-7.6, p<0.0015.

**Table 1: Non-genetic characteristics of the entire French and North American populations.**

| **French population** | | | **USA population** | | | |
|---|---|---|---|---|---|---|
| | Controls | | | Controls | | p |
| | | Cases | | | Cases | |
| **N** | **297** | **1241** | | **1257** | **1732** | |
| | | | | | | |
| **Sex, men, n(%)** | 110 (39.4%) | 415 (33.4%) | ≤0.06 | 543 (43.2%) | 750 (43.3%) | <0.96 |
| **Age, m (sd)*** | 69.2 (7.4) | 78.8 (7.5) | < 0.0001 | 75.0 (5.5) | 80.3 (6.5) | < 0.0001 |
| **Smoking Current, n(%)** | 89 (30.1 %) | 106 (8.6%) | < 0.0001 | 27 (4.6%) | 98 (7.9%) | < 0.005 |
| **Never, n(%)** | 163 (55.1%) | 770 (62.1%) | | 251 (42.9%) | 455 (36.7%) | |
| **Past, n(%)** | 44 (14.8%) | 364 (29.3%) | | 307 (52.5%) | 686 (55.4%) | |

**Table 2: Genotype distributions among the entire French and North American populations.**

| | **French population** | | | **USA population** | | |
|---|---|---|---|---|---|---|
| | **Controls** | **Cases** | | **Controls** | **Cases** | **p** |
| **N** | **297** | **1241** | | **1257** | **1732** | |
| | | | | | | |

| ***CFHY402H (rs1061170)*** | | | | | | |
|---|---|---|---|---|---|---|
| | | | | | | |
| **CC** | 35 (11.8%) | 356 (28.7%) | **< 0.0001** | 154 (12.3%) | 654 (37.8%) | **< 0.0001** |
| **CT** | 146 (49.2%) | 628 (50.6%) | | 562 (44.7%) | 815 (47.1%) | |
| **TT** | 116 (39.0%) | 257 (20.7%) | | 541 (43.0%) | 263 (15.2%) | |

| **ARMS2 (rs1049092 4)** | | | | | | |
|---|---|---|---|---|---|---|
| | | | | | | |
| **GG** | 195 (65.7%) | 397 (32.0%) | **< 0.0001** | 799 (63.6%) | 542 (31.3%) | **< 0.0001** |
| **GT** | 92 (31.0%) | 577 (46.5%) | | 416 (33.1%) | 814 (47.0%) | |
| **TT** | 10 (3.4%) | 267 (21.5%) | | 42 (3.3%) | 376 (21.7%) | |
| | | | | | | |

| **SRB1 (rs5888)** | | | | | | |
|---|---|---|---|---|---|---|
| **CC** | 79 (26.6%) | 317 (25.5%) | **< 0.89** | 376 (29.9%) | 433 (25.0%) | **< 0.004** |
| **CT** | 151 (50.8%) | 629 (50.7%) | | 585 (46.5%) | 903 (52.1%) | |
| **TT** | 67 (22.6%) | 295 (24.8%) | | 296 (23.6%) | 396 (22.9%) | |

| | | | | | | |
|---|---|---|---|---|---|---|
| P values: global chi² test with 2 degrees of freedom for comparison of genotype distribution between cases and controls. | | | | | | |

**Table 3: Non-genetic characteristics of the French and North American populations with no risk alleles for CFH and ARMS2 .**

| | | **French population** | | | **USA population** | | |
|---|---|---|---|---|---|---|---|
| | | | **Cases** | | | **Cases** | **p** |
| | | Controls | | | Controls | | |
| | | | | | | | |

| **N** | | **77** | **61** | | **338** | **85** | |
|---|---|---|---|---|---|---|---|
| | | | | | | | |
| **Sex, men, n(%)** | | 23 (31.9%) | 16 (26.2%) | **< 0.48** | 144 (42.6%) | 39 (45.9%) | **<0.59** |
| **Age, m (sd)*** | | 70.3 (7.2) | 77.9 (9.8) | **< 0.0001** | 74.8 (5.6) | 79.0 (8.0) | **< 0.0001** |
| **Smoking** | | | | | | | |
| | **Current, n(%)** | 19 (24.7%) | 10 (16.4%) | **<0.50** | 8 (5.0%) | 8(14.3%) | **< 0.062^{*}** |
| | **Never, n(%)** | 46 (59.7%) | 41 (67.2%) | | 72 (45.0%) | 20 (35.7%) | |
| | **Past, n(%) 1** | 12 (15.6%) | 10 (16.4%) | | 80 (50.0%) | 28 (50.0%) | |

**Table 4: Adjusted Odds ratio for rs5888 of SCARB1 gene in patients with no risk alleles for CFH and ARMS2**

| | **CC** | **CT** | **TT** | |
|---|---|---|---|---|
| | | OR [CI95%] | OR [CI95%] | **P*** |
| | | | | |
| **France** | 1 (ref) | 3.5 [1.4-8.9] | 1.0 [0.3-3.2] | **< 0.01** |
| **USA** | 1 (ref) | 2.5 [1.1-5.7] | 2.0 [0.8-5.2) | **<0.09** |
| **Pooled** | 1 (ref) | 2.9 [1.6-5.3] | 1.6 [0.8-3.3] | **< 0.002** |
| | | | | |
| **Gender-Pooled** | | | | |
| | 1 (ref) | 4.3 [1.5-11.9] | 1.8 [0.5-6.3] | **< 0.02** |
| **Men** | | | | |
| **Women** | 1 (ref) | 2.5 [1.2-5.5] | 1.7 [0.7-4.3] | **<0.07** |
| | | | | |
| **Exudative forms** | | | | |
| **France** | 1 (ref) | 3.6 [1.3-10.0] | 1.1 [0.3-4.0] | **< 0.02** |
| **USA** | 1 (ref) | 3.5 [1.1-10.5] | 2.2 [0.6-8.0] | **< 0.09** |
| **Pooled** | 1 (ref) | 3.6 [1.7-7.6] | 1.6 [0.7-4.0] | **< 0.0015** |
| | | | | |

Adjustment for non genetic confounders: age, sex, and cigarette smoking;

For table 4, OR are estimated by genotype (CT vs CC and TT vs CT) but the p values are global p values (with 2 degrees of freedom) for estimates a global effect of genotype (is at least one of the genotypes (CT or TT) significantly associated with increased rik of AMD).

*Interaction with center ; p < 0.29, interaction with sex (in pooled population), p < 0.48

Sample size: France : 61 cases and 72 controls - USA : 56 cases and 160 controls

### Discussion:

Here we report for the first time a possible association between a polymorphism in the *SCARB1* gene and AMD, in two distinctly different Caucasian populations.

AMD is a multifactorial disorder including both environmental and genetic factors. Among the long list of genes potentially involved in AMD, two major genes have been recently associated to AMD: *CFH* and *ARMS2.* Because, double homozygosity for the *CFH* and *ARMS2* risk alleles account for more than 50% in the pathology of AMD, we hypothesized that candidate gene screening in a subgroup of AMD patients and controls homozygous for *CFH* and *ARMS2* wild-type alleles may help in identifying novel and independent genetic risk factors. One limit of our study is the sample size of our populations without the CFH and/or the ARMS2 at-risk alleles. Only 61/1241 (4.9%) of AMD French patients and 85/1732 (4.9%) of American AMD did not carry one of these at-risk alleles. Thus, it requires large series of patients in order to assure that some patients without the CFH and/or the ARMS2 at-risk alleles are present in the final sample.

Heterozygosity for the rs5888 SNP of the *SCARB1* gene (CT) may be associated with an increased risk of AMD in the French and North American populations, respectively. Nevertheless, our findings have to be interpreted very cautiously. Heterozygosity was found significantly associated to AMD in wild-type individuals for *CFH* and ARMS2 in the French group (p<0.01), and in the same direction but not significant in the North American population (p<0.09). Heterozygosity at the rs5888 was also found to be significantly associated with AMD when all North American individuals, regardless of their genotypes at the *CFH* and *ARMS2* loci, were considered (p<0.004), but not in the French population. This discrepancy might be explained by the low number of French controls, compared to the North-American sample. However the number of French AMD patients and controls (respectively 1241 and 297) was evaluated in order to obtain similar number of AMD patients and controls in wild-types groups with no risk alleles for CFH and *ARMS2* (respectively 61 and 77). An association between rs5888 of *SCARB1* and the exudative type of AMD was observed (OR: 3.6, 95%CI: 1.7-7.6; p<0015). We enrolled a large number of exudative forms of AMD because patients with neovascular AMD are most often referred to our specialized retina departments than atrophic forms of AMD.

*SCARB1* gene is located in 12q24.31, in a region of interest pointed by linkage analysis. *SCARB1* gene encodes a multiligand cell surface receptor that mediates selective cholesterol uptake, and cholesterol efflux. Reverse cholesterol transport, is a major pathway for the clearance of excess cholesterol from the body. Several studies have reported that the *SCARB1* rs5888 SNP is associated with the development of coronary heart disease, and lipid profile. Indeed, epidemiological studies in Caucasian populations have shown that the rs5888 is associated with increased HDL cholesterol and lower LDL cholesterol, and rs5888 has been reported to be associated with a greater risk of developing coronary heart disease in males. Because AMD and cardiovascular diseases share common pathways, we decided to analyze genes involved in lipid homeostasis. Furthermore, it is known that *SCARB1* is also expressed in the retinal pigment epithelium, and could interact with APOE, another gene which some groups but not all have reported to be involved in AMD. Besides the pathway of lipids, *SCARB1* is also involved in the metabolism of lutein and vitamin E. Lutein, obtained from foods, is a member of the carotenoid family, more specifically the xanthophyll family. Lutein protects the photoreceptors against light-initiated oxidative damage. Furthermore, epidemiologic studies based on diet questionnaires or serum levels of lutein revealed that high levels of lutein are associated with a decreased risk of AMD. Vitamin E acts as an antioxidant, protecting the retina against oxidative stress, with possible preventive and therapeutic effects. *SCARB1* is involved in the metabolism of three key molecules involved in the etiology of AMD: cholesterol, lutein and vitamin E, all supported by fundamental and epidemiological studies. For these reasons based on gene function and gene location, we considered *SCARB1* as a good candidate gene for AMD. The greater risk of AMD found in CT individuals heterozygous at rs5888 has already been reported in peripheral arterial disease. The rs5888 SNP is a coding-synonymous polymorphism (A350A). This polymorphism may nevertheless be in linkage disequilibrium with a functional sequence change as recently demonstrated with the identification of the rs10490924 polymorphism in the *ARMS2* gene which results in instability of the transcript. To evaluate this hypothesis, we sequenced the 13 *SCARB1* exons (in 8 patients: 3 patients TT, 2 patients CC and 3 patients CT) and 200 kb of the 5'UTR in 92 wild-type individuals patients and controls, but we did not found any anomalies in the gene sequence, neither insertions or deletions such as the one described in the *SCARB1* promotor. The relatively small sample size in some of the subgroup analyses could also explain some of the findings. On the other hand, it is also possible that the rs5888 polymorphism has a functional effect through a mechanism involving splicing regulatory system. From this point of view it is worth noting that it has been shown that *SCARB1* mRNA expression is significantly decreased in heterozygous individuals compared to homozygous CC or TT. "A dominant-negative effect can be suggested." Further studies will hopefully bring insights into this intriguing question.

In conclusion, our results suggest that the SCARB1 polymorphism is associated with AMD. This genetic finding is consistent with basic and epidemiological studies underlying the role of cholesterol, lutein and vitamin E in AMD. Additional studies including correlations with serum analysis and larger samples sizes are needed to confirm this finding.

### REFERENCES

Throughout this application, various references describe the state of the art to which this invention pertains.

## Claims

1. A method for predicting risk of age related maculopathy or age-related macular degeneration, in a subject, said method comprising detecting in a sample obtained from said subject the single nucleotide polymorphism rs5888 in the SCARB1 gene wherein the presence of the minor allele T of rs5888 indicates an increased risk for age related maculopathy or age-related macular degeneration.

## Patentansprüche

1. Verfahren zum Vorhersagen eines Risikos für altersbedingte Makulopathie oder altersbedingte Makuladegeneration in einem Individuum, wobei das Verfahren das Detektieren des Einzelnukleotidpolymorphismus rs5888 im SCARB1-Gen in einer von dem Individuum erhaltenen Probe umfasst, wobei das Vorhandensein des Minor-Allels T von rs5888 auf ein erhöhtes Risiko für altersbedingte Makulopathie oder altersbedingte Makuladegeneration hinweist.

## Revendications

1. Procédé de prédiction d'un risque de maculopathie liée à l'âge ou de dégénérescence maculaire liée à l'âge chez un sujet, ledit procédé comprenant la détection dans un échantillon obtenu auprès dudit sujet du polymorphisme mononucléotidique rs5888 dans le gêne SCARB1, dans lequel la présence de l'allèle mineur T du rs5888 indique un risque accru de maculopathie liée à l' âge ou de dégénérescence maculaire liée à l'âge.
